# FASCICULE DE BREVET EUROPEEN

(11) **EP 3 139 349 B1**
(45) Date de publication et mention de la délivrance du brevet: **28.12.2022**
(21) Numéro de dépôt: 15183712.7
(22) Date de dépôt: 03.09.2015
(51) Int. Cl.: G07C 1/22, A63B 24/00

(54) **PROCEDE ET SYSTEME DE MESURE OU DE PREDICTION D'UN TEMPS DE COURSE DE HAIES**
VERFAHREN UND SYSTEM ZUR MESSUNG ODER ZUR VORAUSSAGE EINER ZEIT BEIM HÜRDENLAUF
METHOD AND SYSTEM FOR MEASURING OR PREDICTING A HURDLE RACE TIME

(43) Date de publication de la demande: 08.03.2017
(73) Titulaire: Swiss Timing Ltd., 2606 Corgémont (CH)
(72) Inventeur: Galli, Reto M., 3053 Münchenbuchsee (CH); Richard, Pascal M., 2606 Corgémont (CH)
(74) Mandataire: ICB SA

(56) Documents cités:
- EP-A1- 1 992 389
- EP-A1- 2 654 030
- WO-A1-2011/085501
- CH-A2- 707 401
- Van De Maele Pieter-Jan: "Getting the angular position from gyroscope data | Pieter-Jan.com", , 6 September 2012 (2012-09-06), XP055891720, Retrieved from the Internet: URL:https://www.pieter-jan.com/node/7 [retrieved on 2022-02-15]

## Description

### Domaine de l'invention

L'invention concerne un procédé de mesure ou de prédiction d'un temps de course de haies en athlétisme.

L'invention concerne également un système de mesure ou de prédiction d'un temps de course de haies en athlétisme pour la mise en œuvre du procédé.

### Arrière-plan de l'invention

Dans une course de sprint en athlétisme, telle qu'une course de haies, il est très important d'être bien efficace durant la course notamment lors du passage de chaque haie. Il peut s'agir d'une course de haies sur 60 m, sur 100 m, sur 110 m ou sur 400 m en particulier. Le passage de chaque haie est techniquement difficile et exige une bonne coordination du mouvement des jambes de l'athlète pour être le plus rapide pendant toute la durée de la course. Ainsi lors d'un entraînement, le temps de passage sur chaque haie peut être un paramètre important. Pour ce faire, il existe déjà des haies munies de capteurs par exemple intégrés dans une portion de la haie de manière à détecter le passage de l'athlète. Par simplification, la mesure du temps est effectuée manuellement par les entraîneurs en prenant souvent le temps, lorsqu'un pied de l'athlète touche le sol après le passage de la haie. Il existe des tables avec des temps optimaux au passage de chaque haie pour définir un temps donné à l'arrivée de l'athlète.

La demande de brevet TW 201134517 A décrit un système d'analyse d'une course de haies. Le système comprend plusieurs haies disposées sur une piste en anneau, plusieurs dispositifs de détection et un dispositif d'analyse. Chaque dispositif de détection comprend un capteur d'approche, un capteur de vibration et un capteur tactile. Lorsque l'athlète court et saute une haie, le capteur d'approche envoie un signal de passage. Lorsque l'athlète heurte une haie légèrement, le capteur de vibration envoie un signal de vibration. Lorsque l'athlète heurte fortement la haie, qui tombe, le capteur tactile envoie un signal de contact. Ensuite le dispositif d'analyse peut calculer le temps entre deux haies successives, la vitesse de course et les temps de fautes pour des références d'entraînement. Il n'est nullement prévu de contrôler ou prédire un temps de course au passage de chaque haie de manière à déterminer la position et le temps estimé de chaque athlète en course, ce qui constitue un inconvénient.

Dans une course de haies sur 400 m par exemple, cette course débute dans une courbe, ce qui rend difficile pour les spectateurs de visionner chaque athlète et de savoir quel athlète est en tête de la course avant de les voir sur chaque section droite du tracé de course. Il n'existe pas de manière simple et automatique la possibilité de fournir le temps de passage sur chaque haie en temps réel durant une course. En connaissant ou estimant le temps de passage sur chaque haie, il peut être possible de prédire quel sera le temps final de chaque athlète en course.

Selon l'état de la technique, il n'est pas connu d'estimer ou de prédire un temps de chaque athlète dans une telle course de haies sur la base des temps optimaux notamment après le passage de deux ou trois haies. Avec cela, il serait possible aussi de déterminer une position de chaque athlète afin de connaître quel athlète est en tête de la course notamment d'une course de haies sur 400 m, ce qui est recherché par la présente invention.

La demande de brevet CH 707 401 A2 décrit un procédé pour effectuer la mesure d'un temps dans une compétition sportive d'un compétiteur via un module à transpondeur l'accompagnant durant la compétition. Le module à transpondeur est activé au départ ou dans des positions intermédiaires ou à l'arrivée. Une détection d'une variation de mouvement est effectuée par un capteur de mouvement du module à transpondeur, et transmise à une unité à décodeur du système de mesure pour contrôler un temps de course du compétiteur.

### Résumé de l'invention

L'invention a donc pour but de pallier les inconvénients de l'état de la technique susmentionné en proposant un procédé de mesure ou de prédiction d'un temps de course de haies en athlétisme, dans lequel il est possible d'estimer et prédire le temps final d'une course de haies et de permettre de connaître l'athlète en tête de course simplement.

A cet effet, l'invention concerne un procédé de mesure ou de prédiction d'un temps de course de haies en athlétisme, qui comprend les caractéristiques définies dans les revendications indépendantes 1 et 2.

Des étapes particulières du procédé de mesure ou de prédiction d'un temps de course de haies en athlétisme sont définies dans les revendications dépendantes 3 à 5.

Un avantage du procédé de mesure réside dans le fait qu'avec le module à transpondeur placé sur une partie du corps de l'athlète, il est possible de déterminer ou d'estimer le temps de passage de chaque haie sur le tracé de course en temps réel. Ainsi, il est possible dans la station de base en particulier par réception des signaux de données ou de mesures du module à transpondeur de montrer quel athlète est en tête de la course aux spectateurs et en particulier pour une course de haies sur 400 m.

Avantageusement, le procédé de mesure ou de prédiction permet de prédire le temps d'arrivée de chaque athlète en course sur la base du passage des précédentes haies. Pour des courses de haies de haut niveau, il peut être assumé que les athlètes courent selon un optimum du point de vue de leur régularité de course. Après le passage déjà de deux ou trois haies, on peut déterminer, si l'on peut s'attendre à avoir un record potentiel d'un des athlètes en course.

A cet effet, l'invention concerne aussi un système de mesure ou de prédiction d'un temps de course de haies en athlétisme pour la mise en œuvre du procédé de mesure, et qui comprend les caractéristiques définies dans la revendication indépendante 6.

Des formes d'exécution particulières du système de mesure ou de prédiction d'un temps de course de haies en athlétisme sont définies dans la revendication dépendante 7.

### Brève description des dessins

Les buts, avantages et caractéristiques du procédé et système de mesure ou de prédiction d'un temps de course de haies en athlétisme selon l'invention apparaîtront mieux dans la description suivante d'au moins une forme d'exécution non limitative illustrée par les dessins sur lesquels :
la figure 1 représente schématiquement les principaux éléments d'un système de mesure ou de prédiction d'un temps de course de haies en athlétisme,
la figure 2 représente un athlète en course dans différentes phases de saut d'une haie du procédé de mesure,
la figure 3 représente un graphe dans le temps de l'inclinaison de la partie haute du corps de l'athlète portant le module à transpondeur durant une course de haies sur 100 m du procédé de mesure,
la figure 4 représente de manière schématique un athlète, qui est muni d'un module à transpondeur en position de départ sur un starting-block,
la figure 5 représente de manière schématique différentes positions sur le tracé d'une course de haies de 400 m pour le procédé de mesure, et
la figure 6 représente un graphe de la vitesse de l'athlète entre chaque intervalle d'une course de haies de 400 m, où les références des intervalles sont montrées également en figure 3 d'une course de haies sur 100 m pour le procédé de mesure.

### Description détaillée

Dans la description suivante, tous les éléments du système de mesure ou de prédiction d'un temps de course de haies en athlétisme pour la mise en œuvre du procédé de mesure, qui sont bien connus de l'homme du métier dans ce domaine technique, ne seront relatés que de manière simplifiée.

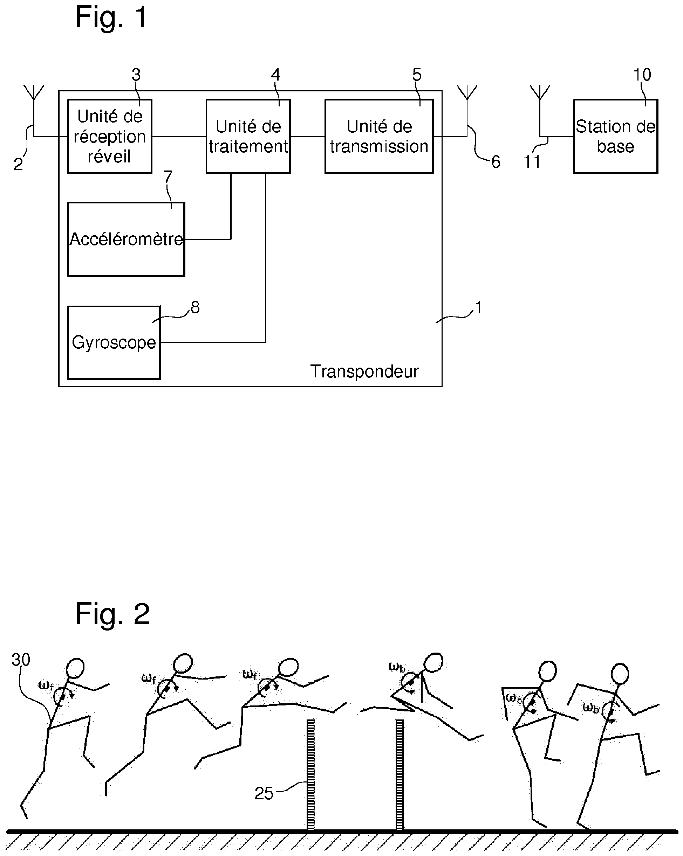

La figure 1 représente schématiquement les principaux éléments, qui composent un système de mesure ou de prédiction d'un temps de course de haies en athlétisme. Pour ce faire, le système comprend un ou plusieurs modules ou circuits à transpondeur 1 et au moins une station de base 10 pour la communication de données et/ou de mesures et/ou de commandes entre les modules ou circuits à transpondeur 1 et la station de base 10. Chaque module à transpondeur 1 pour la compétition est disposé sur une partie du corps d'un athlète, par exemple dans un dossard à numéro, et est donc personnalisé à l'athlète, qui le porte. Le module à transpondeur 1 est disposé sur une partie haute du corps de l'athlète au niveau de son centre gravité, tel que le thorax, de manière à détecter la rotation du haut du corps de l'athlète pour la détermination et prédiction d'un temps de course de haies.

Le module à transpondeur 1 peut être du type actif avec une batterie ou cellule solaire intégrée dans le module ou du type passif en étant alimenté par la réception d'un signal d'interrogation traditionnel.

Le module ou circuit à transpondeur 1 comprend une unité de réception sans fil de signaux 3 pour recevoir par une antenne 2 des signaux de données ou commandes 3 provenant d'une station de base 10 ou d'un émetteur disposé dans un starting-block du système de mesure ou le long d'un tracé de course. La station de base 10 peut être le système de chronométrage de la course et comprend une antenne 11 de transmission ou réception de signaux. Les signaux reçus par l'antenne 2 liée à l'unité de réception 3 sont des signaux permettant le réveil du module à transpondeur 1, qui est dans un état de repos avant la réception de tels signaux. Ces signaux de réveil sont générés, comme indiqué ci-dessus, par la station de base 10 ou par un émetteur du starting-block ou le long du tracé de course. Ces signaux de réveil sont générés par exemple après le signalement de préparation de départ d'une course d'athlétisme notamment ou directement au moment du coup de pistolet de départ. Le pistolet peut être un pistolet électronique ou à poudre avec un transducteur et peut faire partie aussi du système de mesure. Ces signaux de réveil peuvent encore être générés après le départ de la course avant le passage d'une première haie, mais dans ce cas, il peut être nécessaire d'avoir une synchronisation du module à transpondeur 1.

Le module à transpondeur 1 comprend encore une unité de traitement 4, qui peut être une machine d'état, un processeur ou un microcontrôleur, pour la gestion de toutes les données ou commandes ou mesures à recevoir ou à transmettre. L'unité de traitement 4 reçoit les données ou commandes mises en forme dans l'unité de réception 3 de manière également à réveiller tous les composants, qui composent le module à transpondeur 1. L'unité de traitement 4 est encore reliée à une unité de transmission de signaux 5 par une antenne 6 à destination de la station de base 10, qui peut être le système de chronométrage.

Le module à transpondeur 1 comprend encore au moins un capteur de mouvement 7, 8 lié à l'unité de traitement 4 pour fournir des signaux de mesure en continu ou par intermittence à l'unité de traitement 4 une fois que le module à transpondeur est réveillé. Le module à transpondeur 1 comprend comme capteur de mouvement un accéléromètre 7 et/ou un gyromètre ou gyroscope 8. Selon l'invention, il est prévu un accéléromètre 7 pour mesurer l'accélération ou des variations de mouvement d'un athlète au passage de chaque haie de la course et un gyroscope 8 pour déterminer une vitesse ou un sens de rotation et un angle de rotation de la partie haute du corps de l'athlète. Avec cette mesure de rotation du module à transpondeur 1 placé sur la partie haute du corps de l'athlète, il est possible de déterminer précisément le passage de chaque haie, car l'angle de rotation avant et après le passage de la haie est l'inverse. Les signaux de mesure sont fournis directement à l'unité de traitement 4.

L'accéléromètre 7 utilisé est un accéléromètre à un, deux ou trois axes de mesure pour fournir un signal de mesure relatif à une ou plusieurs variations de mouvement dudit module durant la course et au passage des haies. Ces variations de mouvement sont relatives aux chocs de réception d'un pied d'un athlète sur le sol après passage de chaque haie, ce qui correspond à une accélération verticale. Cela doit être détecté différemment par rapport à l'accélération normale de la foulée de course de chaque athlète. Ainsi au-delà d'un certain seuil d'accélération défini ou de variations de mouvement, il est possible d'utiliser cette mesure de l'accéléromètre pour déterminer le temps de passage de chaque haie précisément. Il peut être aussi tenu compte des variations de mouvement sur une période de mesure en tenant compte du temps de vol au-dessus de chaque haie passée durant la course.

Le gyroscope 8 est aussi un gyroscope à un, deux ou trois axes de mesure et peut constituer un ensemble de détection avec l'accéléromètre pour fournir un signal de mesure relatif à la vitesse ou sens de rotation du haut du corps de l'athlète et l'angle de rotation au passage de chaque haie.

Les signaux de mesure de l'accéléromètre 7 et du gyroscope 8 ou d'autres types de capteurs sont échantillonnés par l'unité de traitement 4. Les signaux de mesure peuvent être transmis directement à la station de base 10 en utilisant l'unité de transmission sans fil 5. Toutefois, les signaux de mesure peuvent être améliorés notamment après filtrage et ensuite mémorisés et/ou envoyés par la suite à la station de base 10 après traitement. Il peut aussi être prévu de traiter les données des différents capteurs et tout événement de détection, tel qu'un saut. Il peut encore être prévu de traiter des caractéristiques de mouvement extraites, telles que la fréquence des pas et transmettre cette information à la station de base 10 en plus des données propres à l'accéléromètre 7 et au gyroscope 8.

Il est à noter que le module à transpondeur 1, qui comprend l'accéléromètre et le gyroscope peut être identique à celui décrit dans la demande de brevet EP 2 747 036 A1 aux paragraphes 32 à 37 en relation à la figure 2 de la demande de brevet.

Une synchronisation du module à transpondeur 1 peut être opérée dès le départ de la course de manière à déterminer des temps de passage de haies suite aux mesures effectuées par les capteurs de mouvement directement dans le module à transpondeur avant transmission de signaux de données à la station de base.

Il est encore à noter que les signaux reçus par l'antenne 2 liée à l'unité de réception 3 peuvent être des signaux à basse fréquence de l'ordre de 125 kHz, alors que les signaux transmis par l'antenne 6 liée à l'unité de transmission 5 peuvent être des signaux UHF à fréquence située entre 300 MHz et 3'000 MHz. Toutefois, il peut être concevable d'avoir un module à transpondeur 1 avec une seule antenne de réception et d'émission commutable pour la réception ou l'émission de signaux de données. Dans ce cas de figure, il est préféré avoir une réception d'au moins un signal de réveil et une émission de signaux de données à une fréquence porteuse similaire avec une modulation des données transmises FSK, BPSK, QPSK ou ON-OFF Keying.

Pour être efficace dans une course de haies, l'athlète doit garder son centre de gravité aussi bas que possible durant chaque saut de haie. Pour ce faire, l'athlète doit se plier vers l'avant durant le saut comme on peut le voir sur les différentes positions de l'athlète 30 pour le passage d'une haie 25 sur la figure 2. Le plus haut point du saut est lorsque l'athlète 30 tire la jambe arrière au-dessus de la haie 25, ainsi il serait aussi au maximum de son fléchissement vers l'avant. Dans un saut proche de l'optimum, cela correspond aussi au moment du passage du centre de gravité sur la haie.

Ainsi le gyroscope comme capteur de mouvement mesure une rotation vers l'avant ω_{f} avant que l'athlète 30 passe la haie 25 comme montré par les trois premières positions de l'athlète 30 au passage de la haie 25 et une rotation vers l'arrière ω_{b} après le passage de la haie 25 avec les trois secondes positions de l'athlète 30, qui suivent les trois premières positions. Avec ce changement de signe du sens de rotation au passage au-dessus de chaque haie, il est possible de déterminer un temps de passage par ce changement de signe des signaux de mesure fournis par le gyroscope du module à transpondeur.

Dans le signal brut du gyroscope, le changement de signe de la rotation peut être utilisé pour définir le passage exact sur la haie 25 de chaque athlète 30 en course. Si le signal du gyroscope est intégré pour obtenir l'angle absolu de l'inclinaison de la partie haute du corps de l'athlète 30, l'angle maximum définit le passage de la haie.

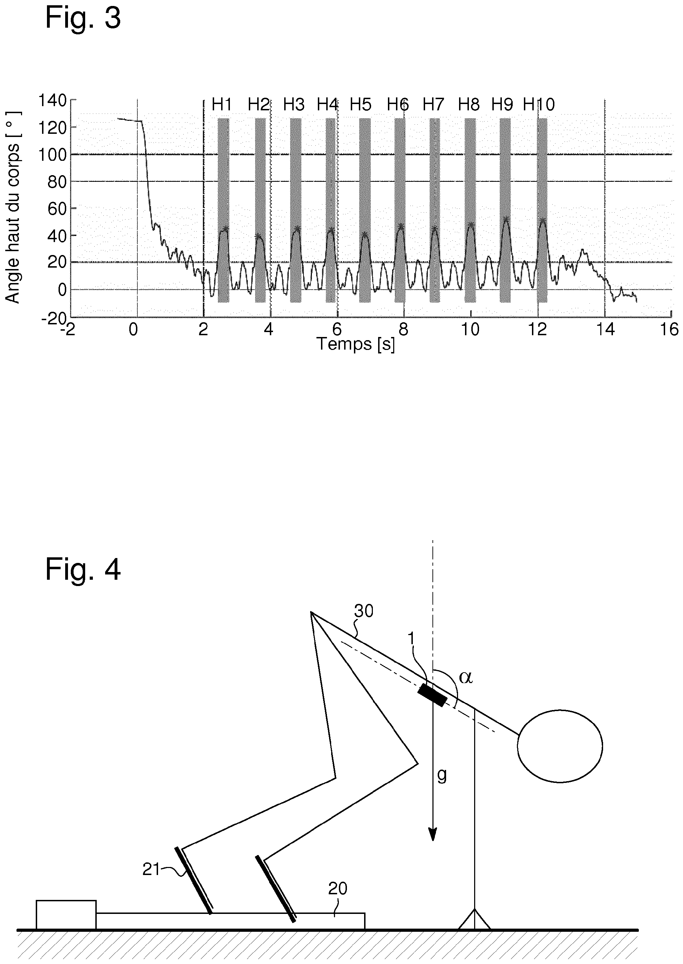

A la figure 3, on peut remarquer l'inclinaison de la partie haute du corps de l'athlète avec des maximums directement au-dessus de chaque haie pour déterminer le passage de chaque haie. Les maximums sont représentés dans chaque rectangle gris en figure 3 avec les indications H1, H2, H3, H4, H5, H6, H7, H8, H9 et H10 des haies de la course. Selon ce graphe, il s'agit d'une course de haies sur 100 m.

Pour faire une estimation de l'inclinaison de la partie haute du corps de l'athlète comme montré à la figure 3, on a besoin de connaître l'angle initial au départ de la course. Cet angle α peut être estimé en mesurant le vecteur de gravité g durant la phase de préparation de l'athlète dans une position accroupie sur le starting-block 20 par exemple comme montré à la figure 4. Il est tenu compte aussi du temps de la commande de préparation du juge de départ et le départ signalé par le coup de pistolet.

La figure 4 représente donc schématiquement l'athlète 30 au moment du départ de la course de haies. Les deux pieds de l'athlète 30 sont en appui contre deux plots d'appui 21 d'un starting-block 20 disposé et fixé sur le sol du tracé de course. L'athlète 30 est équipé d'un module à transpondeur 1 de préférence du type actif. Le module à transpondeur 1 est muni d'au moins un capteur de mouvement, et de préférence de deux capteurs de mouvement, tels que l'accéléromètre et le gyroscope.

Des tests avec des athlètes montrent qu'une précision de l'ordre de ±0.02 s peut être atteinte pour le temps de passage sur chaque haie. Une plus haute précision est atteinte avec le temps au contact de la jambe sur le sol après passage de la haie, c'est-à-dire dès qu'un des pieds touche le sol après la haie. Ceci peut être facilement détecté en évaluant l'accélération verticale causée par le contact au sol après le passage de la haie et en étant mesuré par l'accéléromètre du module à transpondeur. Le contact au sol à l'atterrissage d'un saut de haie peut être distingué d'un contact au sol à chaque enjambée ou foulée de course normale par le temps de vol, qui précède le contact au sol. Bien que la mesure du temps pour le contact au sol est plus précise que la mesure du temps de passage sur la haie, ce n'est pas mieux adapté pour définir un classement, car la position du contact au sol peut varier entre les athlètes.

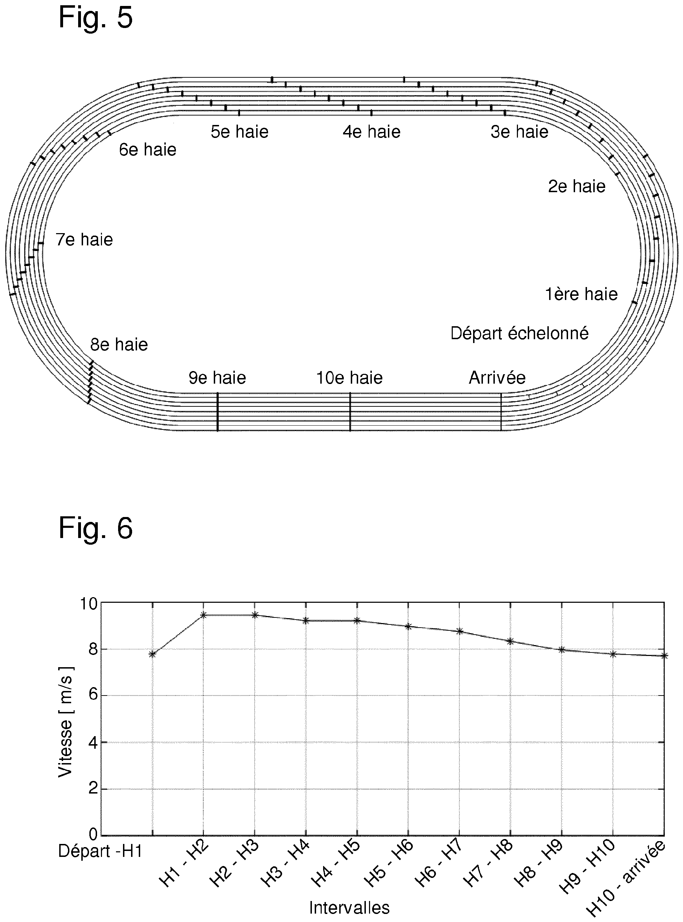

Pour des applications, qui utilisent le temps au passage des haies, les huit premières haies sur la figure 5 dans une course de haies sur 400 m sont échelonnées. Les distances entre le départ et la première haie et entre la dernière haie et l'arrivée sont égales, et ceci sur toutes les lignes de course. Avec cet échelonnement de placement des haies, il est très difficile de voir, qui est en tête d'une telle course. De plus, il n'y a pas de manière simple de créer un classement automatique avant que les athlètes soient sur la partie droite du tracé avant l'arrivée. Selon le procédé de l'invention en ayant le temps sur chaque haie ou le temps de contact au sol après chaque haie, il est possible de créer un classement en temps réel et montrer cette information sur un écran aux spectateurs.

La distance entre deux haies successives est exactement définie dans les règles de compétition internationale pour chaque type de course. Il peut y avoir 8.5 m pour une course de haies sur 100 m, 9.14 m sur 110 m et 35 m sur 400 m. En déterminant le temps au passage de chaque haie, il est possible de calculer la vitesse moyenne pour chaque segment de course, c'est-à-dire entre les haies de la course et créer un profil de vitesse. La figure 6 représente justement une représentation d'un tel profil de vitesse pour une course de haies sur 400 m. Les mêmes indications H1, H2, H3, H4, H5, H6, H7, H8, H9 et H10 de la course de haies sur 100 m décrites en référence à la figure 3 sont reprises sur le graphique de la figure 6 pour définir les intervalles de course. Il est ainsi défini sur le graphe de cette figure 6, une vitesse pour chaque intervalle de course.

Dans une course de haies sur 100 m ou 110 m, un athlète effectue 3 pas entre deux haies successives. C'est seulement possible avec un rythme stable dans un modèle de mouvement. L'accélération entre les haies est dans ces conditions difficilement possible. Des athlètes élites de course de haies s'entraînent pour garder le rythme, ainsi ils ne ralentissent pas. Une telle course de haies consiste en une phase d'accélération entre le départ et la première haie, ensuite une vitesse plus ou moins constante sur toutes les haies est établie et peut être à la fin une autre petite accélération entre la dernière haie et l'arrivée. Ainsi en ayant le temps sur les 4 premières haies passées, il est possible de prédire le temps sur la dernière haie, tout à fait précisément à ±0.05 s en utilisant une extrapolation linéaire. L'accélération possible après la dernière haie peut ne pas changer le temps final d'un athlète élite avec une erreur de +0.05s/-0.1s, mais avec la condition que l'athlète termine la course d'une manière normale, c'est-à-dire que l'athlète ne trébuche pas par exemple. Cela permettrait d'annoncer un potentiel nouveau record sur l'écran des résultats, l'écran TV ou aux commentateurs avant que la course se termine.

A partir de la description qui vient d'être faite, plusieurs variantes du procédé et système de mesure ou de prédiction d'un temps de course de haies en athlétisme peuvent être conçues par l'homme du métier. Le module à transpondeur peut comprendre plusieurs autres capteurs, tels qu'un capteur de température par exemple. Un ou plusieurs modules à transpondeur peuvent être disposés en d'autres endroits que le haut du corps de l'athlète.

## Revendications

1. Procédé de mesure ou de prédiction d'un temps dans une course de haies d'au moins un athlète (30) par l'intermédiaire d'un module à transpondeur (1) personnalisé et disposé sur une partie haute du corps de l'athlète (30), et d'une station de base (10) d'un système de mesure, le module à transpondeur (1) comprenant au moins une unité de réception (3) de signaux, une unité de traitement (4) de données, de mesures ou de commandes, l'unité de traitement étant un microcontrôleur (4), une unité de transmission (5) de signaux de données et/ou de mesures et/ou de commandes, et au moins un capteur de mouvement (7, 8) pour fournir des signaux de mesure au microcontrôleur (4), le capteur de mouvement comprenant un gyroscope (8) à un, deux ou trois axes de mesure,
le procédé étant **caractérisé en ce qu'**il comprend des étapes de :
- activer le module à transpondeur personnalisé (1) suite à la réception d'un signal de réveil dans l'unité de réception (3),
- mesurer, par le gyroscope (8), une vitesse ou un sens de rotation du module à transpondeur (1) disposé sur une partie haute du corps de l'athlète (30), et un angle de rotation de la partie haute du corps de l'athlète (30) au passage d'une ou plusieurs haies durant la course, le gyroscope (8) fournissant ces signaux de mesure au microcontrôleur (4) relatifs à une rotation vers l'avant, avant que l'athlète (30) passe une haie (25) et relatifs à une rotation vers l'arrière, après le passage de la haie (25) définissant un changement de signe du sens de rotation au passage au-dessus de chaque haie ,
- transmettre les signaux de mesure du gyroscope (8) fournis au microcontrôleur (4), par l'unité de transmission (5) à destination de la station de base (10), les signaux de mesure fournis par le gyroscope (8) étant traités et échantillonnés dans le microcontrôleur (4) avant transmission, ou transmettre les signaux de mesure du gyroscope (8) par l'unité de transmission (5) directement à la station de base (10) pour être traités dans la station de base (10) après réception des signaux de mesure, et
- déterminer, dans le microcontrôleur (4) ou dans la station de base (10), un temps de passage sur une ou plusieurs haies en tenant compte d'un changement de signe du sens de rotation dans les signaux de mesure du gyroscope (8).

2. Procédé de mesure ou de prédiction d'un temps dans une course de haies d'au moins un athlète (30) par l'intermédiaire d'un module à transpondeur (1) personnalisé et disposé sur une partie du corps de l'athlète (30), et d'une station de base (10) d'un système de mesure, le module à transpondeur (1) comprenant au moins une unité de réception (3) de signaux, une unité de traitement (4) de données, de mesures ou de commandes, l'unité de traitement étant un microcontrôleur (4), une unité de transmission (5) de signaux de données et/ou de mesures et/ou de commandes, et au moins un capteur de mouvement (7, 8) pour fournir des signaux de mesure au microcontrôleur (4), le capteur de mouvement comprenant un accéléromètre (7) à un, deux ou trois axes de mesure,
le procédé étant **caractérisé en ce qu'**il comprend des étapes de :
- activer le module à transpondeur personnalisé (1) suite à la réception d'un signal de réveil dans l'unité de réception (3),
- mesurer, par l'accéléromètre (7), une ou plusieurs variations d'accélération ou de mouvement du module à transpondeur (1), disposé sur une partie du corps de l'athlète (30) au passage d'une ou plusieurs haies durant la course, l'accéléromètre (7) fournissant au microcontrôleur (4) des signaux de mesure relatifs aux variations de mouvement relatives aux chocs de réception d'un pied d'un athlète (30) sur le sol après passage de chaque haie, correspondant à une accélération verticale,
- transmettre les signaux de mesure de l'accéléromètre (7) fournis au microcontrôleur (4) par l'unité de transmission (5) à destination de la station de base (10), les signaux de mesure fournis par l'accéléromètre (7) étant traités et échantillonnés dans le microcontrôleur (4) avant transmission, ou transmettre les signaux de mesure de l'accéléromètre (7) par l'unité de transmission (5) directement à la station de base (10) pour être traités dans la station de base (10) après réception des signaux de mesure, et
- déterminer, dans le microcontrôleur (4) ou dans la station de base (10), un temps de passage sur une ou plusieurs haies en utilisant l'accélération verticale au-delà d'un seuil d'accélération prédéfini dans les signaux de mesure de l'accéléromètre (7).

3. Procédé de mesure selon l'une des revendications 1 et 2, **caractérisé en ce que** le signal de réveil est reçu par l'unité de réception (3) du module à transpondeur (1) pour l'activer en provenance de la station de base (10) ou d'un émetteur d'un point de départ de course ou d'un point du tracé de course de l'athlète (30) au moment d'un signal de départ, généré par un coup de pistolet du système de mesure.

4. Procédé de mesure selon l'une des revendications précédentes, **caractérisé en ce que** suite à la mesure du ou des capteurs de mouvement au passage de deux ou trois haies successives de plusieurs athlètes en course, le microcontrôleur (4) ou la station de base (10) ayant reçu les signaux de mesure déterminent un classement en temps réel des athlètes durant la course.

5. Procédé de mesure selon la revendication 1, **caractérisé en ce qu'**une intégration du signal du gyroscope est effectuée pour obtenir l'angle absolu de l'inclinaison de la partie haute du corps de l'athlète (30), l'angle maximum définissant le passage de la haie.

6. Système de mesure ou de prédiction d'un temps dans une course de haies d'un athlète (30) pour la mise en œuvre du procédé de mesure selon la revendication 1, le système de mesure comprenant au moins un module à transpondeur (1) personnalisé et destiné à être placé sur une partie haute du corps d'un athlète (30), et une station de base (10), ledit module à transpondeur (1) comprenant au moins une unité de réception (3) de signaux, une unité de traitement (4) de données, de mesures ou de commandes, l'unité de traitement étant un microcontrôleur (4), une unité de transmission (5) de signaux de données et/ou de mesures et/ou de commandes, et au moins un capteur de mouvement (7, 8) pour fournir des signaux de mesure au microcontrôleur (4),
**caractérisé en ce que** le module à transpondeur (1) est configuré pour être réveillé par un signal de réveil reçu par l'unité de réception (3) pour que le capteur de mouvement (7, 8), qui comprend un gyroscope (8) à un, deux ou trois axes de mesure relié au microcontrôleur (4), mesure une vitesse ou un sens de rotation du module à transpondeur (1) sur l'athlète (30) au passage d'une ou plusieurs haies durant la course, le gyroscope (8) étant agencé pour mesurer une rotation vers l'avant, avant que l'athlète (30) passe une haie (25) et une rotation vers l'arrière, après le passage de la haie (25) définissant un changement de signe du sens de rotation au passage au-dessus de chaque haie, et
**en ce que** la station de base (10) ou le module à transpondeur (1) sont adaptés pour la détermination d'un temps de passage sur la ou les haies durant la course en tenant compte d'un changement de signe du sens de rotation dans les signaux de mesure du gyroscope (8).

7. Système de mesure selon la revendication 6, **caractérisé en ce que** le module à transpondeur (1) comprend un autre capteur de mouvement, qui est un accéléromètre (7) à un, deux ou trois axes de mesure.

## Patentansprüche

1. Verfahren zur Messung oder zur Vorhersage einer Zeit bei einem Hürdenlauf mindestens eines Athleten (30) anhand eines personalisierten und an einem oberen Teil des Körpers des Athleten (30) angeordneten Transpondermoduls (1) und einer Basisstation (10) eines Mess-Systems, wobei das Transpondermodul (1) mindestens eine Empfangseinheit (3) von Signalen, eine Verarbeitungseinheit (4) von Daten, Messungen oder Steuerungen, wobei die Verarbeitungseinheit ein Mikrocontroller (4) ist, eine Übertragungseinheit (5) von Daten-, Mess- und/oder Steuersignalen und mindestens einen Bewegungssensor (7, 8) umfasst, um dem Mikrocontroller (4) Mess-Signale bereitzustellen, wobei der Bewegungssensor ein Gyroskop (8) mit einer, zwei oder drei Messachsen umfasst,
wobei das Verfahren **dadurch gekennzeichnet ist, dass** es die folgenden Schritte umfasst:
- Aktivieren des personalisierten Transpondermoduls (1) infolge des Empfangs eines Wecksignals in der Empfangseinheit (3),
Messen, durch das Gyroskop (8), einer Geschwindigkeit oder einer Rotationsrichtung des Transpondermoduls (1), das an einem oberen Teil des Körpers des Athleten (30) angebracht ist, und eines Rotationswinkels des oberen Teils des Körpers des Athleten (30) beim Passieren einer oder mehrerer Hürden während des Laufs, wobei das Gyroskop (8) diese Mess-Signale dem Mikrocontroller (4) bereitstellt, die sich auf eine Rotation nach vorn beziehen, bevor der Athlet (30) eine Hürde (25) passiert, und auf eine Rotation nach hinten, nach dem Passieren der Hürde (25), was mit einem Wechsel des Vorzeichens der Rotationsrichtung beim Passieren jeder Hürde einhergeht,
- Übertragen der dem Mikrocontroller (4) bereitgestellten Mess-Signale des Gyroskops (8) durch die Übertragungseinheit (5) in Richtung der Basisstation (10), wobei die vom Gyroskop (8) bereitgestellten Mess-Signale im Mikrocontroller (4) vor der Übertragung verarbeitet und gesampelt werden, oder Übertragen der Mess-Signale des Gyroskops (8) durch die Übertragungseinheit (5) direkt an die Basisstation (10) um in der Basisstation (10) nach Empfang der Mess-Signale verarbeitet zu werden, und
- Bestimmen, im Mikrocontroller (4) oder in der Basisstation (10), einer Passierzeit über eine oder mehrere Hürden unter Berücksichtigung eines Wechsels des Vorzeichens der Rotationsrichtung in den Mess-Signalen des Gyroskops.

2. Verfahren zur Messung oder zur Vorhersage einer Zeit bei einem Hürdenlauf mindestens eines Athleten (30) anhand eines personalisierten und an einem Teil des Körpers des Athleten (30) angeordneten Transpondermoduls (1) und einer Basisstation (10) eines Mess-Systems, wobei das Transpondermodul (1) mindestens eine Empfangseinheit (3) von Signalen, eine Verarbeitungseinheit (4) von Daten, Messungen oder Steuerungen, wobei die Verarbeitungseinheit ein Mikrocontroller (4) ist, eine Übertragungseinheit (5) von Daten-, Mess- und/oder Steuersignalen und mindestens einen Bewegungssensor (7, 8) umfasst, um dem Mikrocontroller (4) Mess-Signale bereitzustellen, wobei der Bewegungssensor einen Beschleunigungsmesser (7) mit einer, zwei oder drei Messachsen umfasst,
wobei das Verfahren **dadurch gekennzeichnet ist, dass** es die folgenden Schritte umfasst:
- Aktivieren des personalisierten Transpondermoduls (10) infolge des Empfangs eines Wecksignals in der Empfangseinheit (3),
Messen, durch den Beschleunigungsmesser (7), einer oder mehrerer Beschleunigungs- oder Bewegungsvariationen des Transpondermoduls (1), das an einem Teil des Körpers des Athleten (30) angebracht ist, beim Passieren einer oder mehrerer Hürden während des Laufs, wobei der Beschleunigungsmesser (7) dem Mikrocontroller (4) Mess-Signale bereitstellt, die sich auf Bewegungsvariationen beziehen, die sich auf Stöße beim Aufsetzen eines Fußes eines Athleten (30) auf dem Boden nach dem Passieren jeder Hürde beziehen, was einer vertikalen Beschleunigung entspricht,
- Übertragen der dem Mikrocontroller (4) bereitgestellten Mess-Signale des Beschleunigungsmessers (7) durch die Übertragungseinheit (5) in Richtung der Basisstation (10), wobei die vom Beschleunigungsmesser (7) bereitgestellten Mess-Signale im Mikrocontroller (4) vor der Übertragung verarbeitet und gesampelt werden, oder Übertragen der Mess-Signale des Beschleunigungsmessers (7) durch die Übertragungseinheit (5) direkt an die Basisstation (10), um in der Basisstation (10) nach Empfang der Mess-Signale verarbeitet zu werden, und
- Bestimmen, im Mikrocontroller (4) oder in der Basisstation (10), einer Passierzeit über eine oder mehrere Hürden bei Verwendung der vertikalen Beschleunigung jenseits einer vorher festgelegten Beschleunigungsschwelle in den Mess-Signalen des Beschleunigungsmessers (7).

3. Messverfahren nach einem der Ansprüche 1 und 2, **dadurch gekennzeichnet, dass** das Wecksignal von der Empfangseinheit (3) des Transpondermoduls (1) von der Basisstation (10) oder einem Sender an einem Ausgangspunkt des Laufs oder an einem Laufwegpunkt des Athleten (30) im Augenblick eines Startsignals, das von einem Pistolenschuss des Mess-Systems erzeugt wird, zwecks Aktivierung empfangen wird.

4. Messverfahren nach einem der vorangehenden Ansprüche, **dadurch gekennzeichnet, dass** der Mikrocontroller (4) oder die Basisstation (10), die die Mess-Signale erhalten haben, nach der Messung durch den oder die Bewegungssensoren beim Passieren von zwei oder drei aufeinanderfolgenden Hürden durch mehrere laufende Athleten eine Klassifizierung der Athleten während des Laufs in Echtzeit vornehmen.

5. Messverfahren nach Anspruch 1, **dadurch gekennzeichnet, dass** eine Integration des Signals des Gyroskops durchgeführt wird, um den absoluten Winkel der Neigung des oberen Teils des Körpers des Athleten (30) zu erhalten, wobei der maximale Winkel dem Passieren der Hürde entspricht.

6. System zum Messen oder Vorhersagen einer Zeit bei einem Hürdenlauf eines Athleten (30) für die Durchführung des Messverfahrens nach Anspruch 1, wobei das Mess-Systems mindestens ein personalisiertes Transpondermodul (1) umfasst, das bestimmt ist, an einem oberen Teil des Körpers eines Athleten (30) platziert zu sein, und eine Basisstation (10), wobei das Transpondermodul (1) mindestens eine Empfangseinheit (3) von Signalen, eine Verarbeitungseinheit (4) von Daten, Messungen oder Steuerungen, wobei die Verarbeitungseinheit ein Mikrocontroller (4) ist, eine Übertragungseinheit (5) von Daten-, Mess- und/oder Steuersignalen und mindestens einen Bewegungssensor (7, 8) umfasst, um dem Mikrocontroller (4) Mess-Signale bereitzustellen,
**dadurch gekennzeichnet, dass** das Transpondermodul (1) ausgelegt ist, um von einem von der Empfangseinheit (3) empfangenen Wecksignal geweckt zu werden, damit der Bewegungssensor (7, 8), der ein Gyroskop (8) mit einer, zwei oder drei Messachsen umfasst, das mit dem Mikrocontroller (4) verbunden ist, eine Geschwindigkeit oder eine Rotationsrichtung des Transpondermoduls (1) an dem Athleten (30) beim Passieren von einer oder mehreren Hürden während des Laufs misst, wobei das Gyroskop (8) eingerichtet ist, um eine Rotation nach vorn zu messen, bevor der Athlet (30) eine Hürde (25) passiert, und nach der Passage der Hürde (25) eine Rotation nach hinten, was mit einem Wechsel des Vorzeichens der Rotationsrichtung beim Passieren jeder Hürde einhergeht, und
dass die Basisstation (10) oder das Transpondermodul (1) für die Bestimmung einer Passierzeit über die Hürde oder Hürden während des Laufs unter Berücksichtigung eines Wechsels des Vorzeichens der Rotationsrichtung der Mess-Signale des Gyroskops (8) geeignet sind.

7. Mess-System nach Anspruch 6, **dadurch gekennzeichnet, dass** das Transpondermodul (1) einen anderen Bewegungssensor umfasst, der ein Beschleunigungsmesser (7) mit einer, zwei oder drei Messachsen ist.

## Claims

1. Process for measuring or predicting a time in a hurdle race of at least one athlete (30) by means of a personalised transponder module (1) positioned on an upper part of the body of the athlete (30), and a base station (10) of a measurement system, the transponder module (1) comprising at least a signal receiver unit (3), a processing unit (4) for data, measurements or commands, the processing unit being a microcontroller (4), a transmitter unit (5) for data and/or measurement and/or command signals, and at least one motion sensor (7, 8) to supply measurement signals to the microcontroller (4), the motion sensor comprising a gyroscope (8) with one, two or three measurement axes,
the process being **characterised in that** it comprises steps of:
- activating the personalised transponder module (1) following the receipt of a wake-up signal in the receiver unit (3),
- measuring, with the gyroscope (8), a speed or a direction of rotation of the transponder module (1) positioned on an upper part of the body of the athlete (30), and an angle of rotation of the upper part of the body of the athlete (30) on passage of one or more hurdles during the race, the gyroscope (8) supplying these measurement signals to the microcontroller (4) relative to a forward rotation, before the athlete (30) passes a hurdle (25) and relative to a backward rotation, after the passage of the hurdle (25) defining a change of sign of the direction of rotation on the passage over each hurdle,
- transmitting the measurement signals from the gyroscope (8) supplied to the microcontroller (4), by the transmission unit (5) to the base station (10), the measurement signals supplied by the gyroscope (8) being processed and sampled in the microcontroller (4) before transmission, or transmitting the measurement signals from the gyroscope (8) by the transmission unit (5) directly to the base station (10) to be processed in the base station (10) after receiving the measurement signals, and
- determining, in the microcontroller (4) or in the base station (10), a passage time over one or more hurdles taking into consideration a change of sign of the direction of rotation in the measurement signals from the gyroscope (8).

2. Process for measuring or predicting a time in a hurdle race of at least one athlete (30) by means of a personalised transponder module (1) positioned on an upper part of the body of the athlete (30) and a base station (10) of a measurement system, the transponder module (1) comprising at least a signal receiver unit (3), a processing unit (4) for data, measurements or commands, the processing unit being a microcontroller (4), a transmitter unit (5) for data and/or measurement and/or command signals, and at least one motion sensor (7, 8) to supply measurement signals to the microcontroller (4), the motion sensor comprising an accelerometer (7) with one, two or three measurement axes,
the process being **characterised in that** it comprises steps of:
- activating the personalised transponder module (1) following the receipt of a wake-up signal in the receiver unit (3),
- measuring, with the accelerometer (7), one or more variations in acceleration or movement of the transponder module (1), positioned on an upper part of the body of the athlete (30) on passage of one or more hurdles during the race, the accelerometer (7) supplying measurement signals to the microcontroller (4) relative to the variations in movement relative to the landing shocks of a foot of an athlete (30) on the ground after passing each hurdle, corresponding to a vertical acceleration,
- transmitting the measurement signals from the accelerometer (7) supplied to the microcontroller (4), by the transmission unit (5) to the base station (10), the measurement signals supplied by the accelerometer (7) being processed and sampled in the microcontroller (4) before transmission, or transmitting the measurement signals from the accelerometer (7) by the transmission unit (5) directly to the base station (10) to be processed in the base station (10) after receiving the measurement signals, and
- determining, in the microcontroller (4) or in the base station (10), a passage time over one or more hurdles using the vertical acceleration beyond a predefined acceleration threshold in the measurement signals from the accelerometer (7).

3. Measurement process according to one of claims 1 and 2, **characterised in that** the wake-up signal is received by the receiver unit (3) of the transponder module (1) to activate it from the base station (10) or an emitter of a race starting point or of a point of the race track of the athlete (30) at the instant of a starting signal, generated by a starting gun shot of the measurement system.

4. Measurement process according to one of the preceding claims, **characterised in that** following the measurement of the motion sensor or sensors on passage of two or three successive hurdles of several racing athletes, the microcontroller (4) or the base station (10) having received the measurement signals determines a placing of the athletes in real time during the race.

5. Measurement process according to claim 1, **characterised in that** the signal from the gyroscope is integrated to obtain the absolute angle of inclination of the upper part of the body of the athlete (30), the maximum angle defining the passage of the hurdle.

6. System for measuring or predicting a time in a hurdle race of an athlete (30) for implementing the measurement process according to claim 1, the measurement system comprising at least one personalised transponder module (1) intended to be positioned on an upper part of the body of an athlete (30), and a base station (10), said transponder module (1) comprising at least a signal receiver unit (3), a processing unit (4) for data, measurements or commands, the processing unit being a microcontroller (4), a transmitter unit (5) for data and/or measurement and/or command signals, and at least one motion sensor (7, 8) to supply measurement signals to the microcontroller (4),
**characterised in that** the transponder module (1) is configured to be woken up by a wake-up signal received by the receiver unit (3) so that the motion sensor (7, 8), which comprises a gyroscope (8) with one, two or three measurement axes connected to the microcontroller (4), measures a speed or a direction of rotation of the transponder module (1) on the athlete (30) on passage of one or more hurdles during the race, the gyroscope (8) being arranged to measure a forward rotation, before the athlete (30) passes a hurdle (25) and a backward rotation, after the passage of the hurdle (25) defining a change of sign of the direction of rotation on the passage over each hurdle, and
and **in that** the base station (10) or the transponder module (1) is adapted to determine a passage time over the hurdle or hurdles during the race taking into consideration a change of sign of the direction of rotation in the measurement signals from the gyroscope (8).

7. Measurement system according to claim 6, **characterised in that** the transponder module (1) comprises another motion sensor, which is an accelerometer (7) with one, two or three measurement axes.
